# EUROPEAN PATENT APPLICATION

(11) **EP 3 954 330 A1**
(43) Date of publication of application: **16.02.2022**
(21) Application number: 20787930.5
(22) Date of filing: 03.04.2020
(51) Int. Cl.: A61F 2/24

(54) **CONNECTING STRUCTURE FOR STENT AND VALVE LEAFLETS, AND INTERVENTIONAL PULMONARY VALVE AND INTERVENTIONAL AORTIC VALVE USING SAME**

(30) Priority: 08.04.2019 CN 201910274554
(71) Applicant: Beijing Balance Medical Technology Co., Ltd., Beijing 102200 (CN)
(72) Inventor: JIN, Lei, Beijing 102200 (CN); MU, Hong, Beijing 102200 (CN); FAN, Zhihao, Beijing 102200 (CN)
(74) Representative: Murgitroyd & Company
(86) International application number: PCT/CN2020/083087
(87) International publication number: WO 2020/207331

(57) **Abstract**

The invention relates to a connecting structure of a stent and a valve leaflet for an interventional aortic valve or an interventional pulmonary valve, wherein the stent is a metal mesh tube, the valve leaflets are three fan-shaped valve leaflets arranged on the inner side of the stent, each of the three fan-shaped valve leaflets is provided with a free edge, an arc-shaped bottom edge and valve leaflet junction connecting parts extending on two sides, three connecting posts are uniformly distributed on the metal mesh tube, and the junction connecting parts on the two sides of each valve leaflet are folded on the inner side of each connecting post to form a cushioning portion, and then are connected and fixed to the connecting posts through sutures. The invention also provides an interventional pulmonary valve and interventional aortic valve applying the connecting structure. The connecting structure of the stent and the valve leaflets can avoid stress concentration when the valve leaflets are opened and closed and friction generated between the stent and the valve leaflets, so that hemodynamic effects similar to bioprosthetic valves and similar durability functions are realized.

## Description

### Technical Field

The invention relates to the technical field of medical instruments, in particular to a connecting structure of a stent and a valve leaflet for interventional pulmonary valves and interventional aortic valves, and an interventional pulmonary valve and an interventional aortic valve applying the connecting structure.

### Background Art

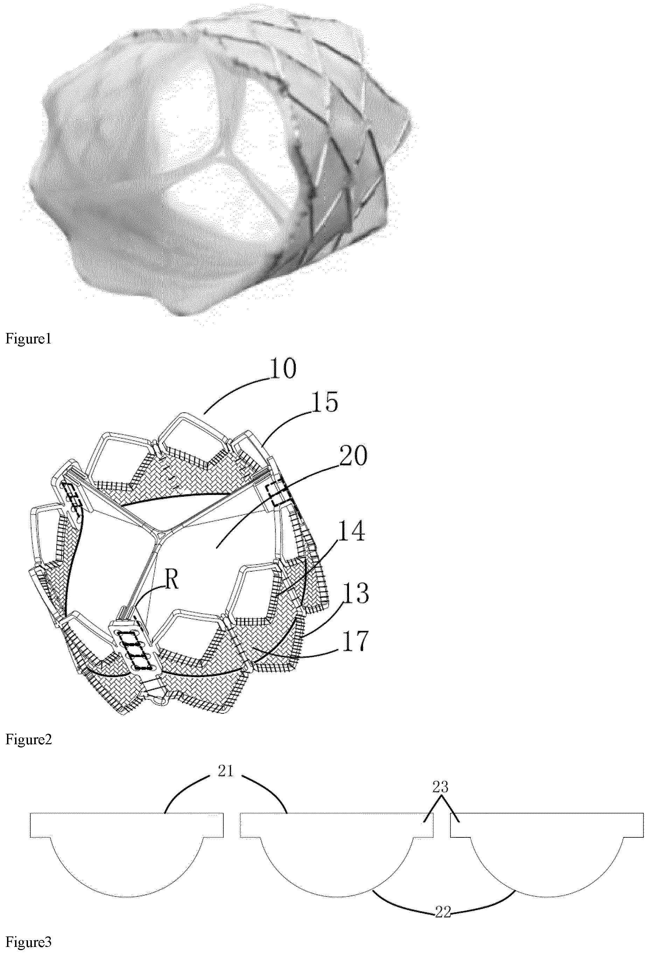

The first report of success in transcatheter pulmonary valve intervention was in 2000, a valve known as Melody^{™} transcatheter pulmonary valve (shown in FIG. 1). Subsequently, it was approved by FDA Humanitarian Exemption Device Approval (HDE) in 2010 and approved again by PMA application in 2015, which became officially used for interventional therapy of pulmonary valve dysfunction (valve stenosis and/or insufficiency) after right ventricular outflow tract reconstruction in complex congenital heart disease. The bioprosthetic valve tissue is derived from bovine jugular vein valve. Subsequently, the application of transcatheter aortic valve (TAVR) has been successful. After nearly 20 years of development, the interventional valve has become mature for the treatment of elderly patients with aortic valve.

China is a large country with nearly 1.4 billion people. There are nearly 1 million children with birth defects each year, involving pulmonary valve birth defects, belonging to complex congenital heart disease (referred to as complex CHD). According to "*Report on Cardiovascular Disease in China (2017)*", 200 million patients with congenital heart disease in China, about 1/5 of them have complex congenital heart disease. For most of these children, surgical reconstruction of the pulmonary valve and the main pulmonary artery is the only effective treatment. In order to solve this problem, the applicant has a patent entitled "Artificial Pulmonary Artery Valved Duct (ZL 200710064337.9)" for cardiac surgery implantation to reconstruct the right ventricular outflow tract and pulmonary valve, which was approved for registration and marketing in December 2016. Similar inventions include "valved patches for cardiac outflow tract" (ZL 200510082673.7), "valved patches and valved conduits for repair of cardiac outflow tract (US 11/995, 106)" and "stentless artificial bioprosthetic valve (ZL 200510082674.1)".

In view of the problem of durability which is generally concerned with the clinical application of the interventional valve at present, the current research on the durability of the interventional pulmonary valve in the industry patent is still in the initial stage.

### Summary of the Invention

In view of this, the technical problem to be solved by the present invention is to provide a connecting structure of a stent and valve leaflet for interventional pulmonary valves and interventional aortic valves, and an interventional pulmonary valve and an interventional aortic valve using the connecting structure, wherein a cushioning portion is arranged on the inner side of a metal stent, so that the valve leaflets can be prevented from being in direct contact with the stent, and the service durability of a valve product is improved.

In order to solve the technical problem, the technical scheme adopted by the invention is to provide a connecting structure of a stent and valve leaflets for interventional pulmonary valves and interventional aortic valves, wherein the stent is a metal mesh tube, the valve leaflets are three fan-shaped valve leaflets arranged on the inner side of the stent, each of the three fan-shaped valve leaflets is provided with a free edge, an arc-shaped bottom edge and valve leaflet junction connecting parts extending on two sides, and three connecting posts are uniformly distributed on the metal mesh tube; and the junction connecting parts on the two sides of each valve leaflet are folded on the inner side of each connecting post to form a cushioning portion, and then are connected and fixed to the connecting posts through sutures.

Furthermore, double columns of holes or double columns of rectangular frames are arranged on each connecting post, the number of the holes is four to eight, and the number of the rectangular frames is two or four.

Furthermore, the junction connecting part is folded once or twice to form the cushioning portion and connected and fixed to the hole or the rectangular frame of the connecting post through sutures.

Furthermore, the material of the stent is an implantable alloy material, the implantable alloy material is a cobalt-based alloy, a nickel-titanium alloy or a stainless steel material, and the material of the valve leaflet is an animal-derived tissue material or a medical polymer material. The invention also provides an interventional pulmonary valve applying the above connecting structure, which comprises a stent being radially compressible and self-expandable with two ends in a uniform cylindrical flaring shape, three fan-shaped valve leaflets arranged on the inner side of the stent, the three fan-shaped valve leaflets are respectively provided with a free edge, an arc-shaped bottom edge and valve leaflet junction connecting parts extending on the two sides, the stent is a metal mesh tube, and three connecting posts are uniformly distributed on the metal mesh tube; and the junction connecting parts on the two sides of each valve leaflet are folded on the inner side of each connecting post to form a cushioning portion, then are connected and fixed to the connecting posts through sutures, and a coating membrane is arranged on the wall body of the stent.

Furthermore, double columns of holes or double columns of rectangular frames are arranged on each connecting post, the number of the holes is four to eight, and the number of the rectangular frames is two or four. Further, the junction connecting part is folded once or twice to form the cushioning portion and connected and fixed to the hole or the rectangular frame of the connecting post through sutures. Furthermore, the material of the stent is an implantable alloy material, the implantable alloy material is a cobalt-based alloy, a nickel-titanium alloy or a stainless steel material, and the material of the valve leaflet is an animal-derived tissue material or a medical polymer material.

Furthermore, the stent is provided with a plurality of columns of axial supporting rods arranged between the connecting posts, six rows of transversely extending circumferential supporting rods are arranged between the connecting posts and the axial supporting rods, the lower first, second and third rows of circumferential supporting rods define the inflow end of the stent, the fourth, fifth and sixth rows of circumferential supporting rods define the outflow end of the stent, and each row of circumferential supporting rods consists of a plurality of groups of angular supporting rods connected together; each group of supporting rods is in the shape of a deformable V, the deformation angle is 0-90 degrees, each row of circumferential supporting rods and a plurality of columns of axial supporting rods form a plurality of rows of rhombic or honeycomb grids, and a coating membrane on the body wall of the stent is sewn to the grids in the middle row of the stent. Further, the angle between the outer edge of the balloon-expanded stent and its axis is between 0° and 30°.

The invention also provides an interventional aortic valve applying the above connecting structure, which comprises a sent that can be radially compressible and in a slightly flaring shape after being expanded by a balloon, three fan-shaped valve leaflets arranged on the inner side of the stent, the three fan-shaped valve leaflets are respectively provided with a free edge, an arc-shaped bottom edge and valve leaflet junction connecting parts extending on the two sides, the stent is a metal mesh tube, and three connecting posts are uniformly distributed on the metal mesh tube; and the junction connecting parts on the two sides of each valve leaflet are folded on the inner side of each connecting post to form a cushioning portion, then are connected and fixed to each of the connecting posts through sutures, and a coating membrane is arranged on the body wall of the stent.

Furthermore, double columns of holes or double columns of rectangular frames are arranged on each connecting post, the number of the holes is four to eight, and the number of the rectangular frames is two or four. Further, the junction connecting part is folded once or twice to form the cushioning portion and connected and fixed to the hole or the rectangular frame of the connecting post through sutures. Furthermore, the material of the stent is an implantable alloy material, the implantable alloy material is a cobalt-based alloy, a nickel-titanium alloy or a stainless steel material, and the material of the valve leaflet is an animal-derived tissue material or a medical polymer material.

Furthermore, the stent is provided with a plurality of columns of axial supporting rods arranged between the connecting posts, three rows of transversely extending circumferential supporting rods are arranged between the connecting posts and the axial supporting rods, the lower first row of circumferential supporting rods define the inflow end of the stent, the second row of circumferential supporting rods and the third row of circumferential supporting rods spaced from the first row define an outflow end of the stent, and each row of circumferential supporting rods consists of a plurality of groups of angular supporting rods connected together; each group of supporting rods is in the shape of a deformable V, the deformation angle is 0-90 degrees, each group of circumferential supporting rods in the first row and the second row are arranged in parallel and opposite to the direction of each group of circumferential supporting rods in the third row, and a coating membrane on the body wall of the stent is sewn between the first row of circumferential supporting rods and the second row of circumferential supporting rods. Furthermore, the stent is provided with four rows of transversely extending circumferential supporting rods and a plurality of columns of axial supporting rods arranged between the circumferential supporting rods, wherein the lower first and second row of circumferential supporting rods define the inflow end of the stent, the third and fourth row of circumferential supporting rods define the outflow end of the stent, each row of circumferential supporting rods consists of a plurality of groups of angular supporting rods connected together, and each group of supporting rods is in a deformable V shape; the deformation angle is 0-90 degrees, a plurality of columns of axial supporting rods and a plurality of groups of circumferential supporting rods are mutually connected to form a honeycomb space, and a coating membrane on the body wall of the stent is sewn between the first row of circumferential supporting rods and the third row of circumferential supporting rods.

Further, a coating membrane is sewn between the first row of circumferential supporting rods and the second row of circumferential supporting rods outside the body wall of the stent.

Further, the angle between the outer edge of the balloon-expanded stent and its axis is between 0° and 30°.

The invention has the beneficial effects that: the invention provides a connecting structure of a stent and valve leaflets for interventional pulmonary valves and interventional aortic valves, and an interventional aortic valve and an interventional pulmonary valve applying the connecting structure. Due to the fact that the animal-derived tissue material is folded at the inner side of the valve leaflet junction connection tissue to form a cushioning portion, and then the cushioning portion is connected and fixed on the connecting post of the stent through sutures, the valve leaflets can be prevented from being rubbed or scratched by the metal stent in the opening and closing processes; and due to the fact that the connecting posts in the connecting structure are double rows of holes or rectangular frame routing, the sutures at the seams of the junction connecting parts of the valve leaflets are fully fixed with the holes or the rectangular frame, stress concentration of the valve leaflets in the opening and closing processes can be avoided, use durability of the interventional aortic valve or the interventional pulmonary valve is improved, and a durable effect equivalent to that of a surgical valve is achieved. On the basis of the original product, the artificial biological pulmonary valve is implanted in a minimally invasive catheter-passing mode in the pulmonary valve of the present application, and particularly, for adult patients with complex cardiac surgery who have been operated to reconstruct the right ventricular outflow tract for many years, they can be treated without another thoracic surgery. The invention aims to design a valve stent, in particular to a connecting structure of the stent and a valve leaflet, and based on the experience of successful application of a surgical bioprosthetic valve previously, the connection and fixation of the valve leaflet and the stent are key points of firmness and durability of an interventional artificial bioprosthetic valve. In this manner, an interventional pulmonary valve having hemodynamic characteristics and durability consistent with the surgical artificial bioprosthetic valve of the enterprise will be obtained by the present invention. Meanwhile, the invention can also be used for sewing an interventional aortic valve.

### Brief Description of the Drawings

FIG. 1 is a schematic view of interventional pulmonary valve structure in prior art;
FIG. 2 is a schematic view of an interventional aortic valve structure for interventional therapy with an artificial heart valve according to an example of the present invention;
FIG. 3 is a valve leaflet deployment plan view of an interventional aortic valve for interventional therapy of an artificial bioprosthetic heart valve according to an example of the present invention;
FIGS. 4A and 4B are side and perspective views of the folding suture of three valve leaflets at junction connecting part commissures of an interventional aortic valve for interventional therapy of an artificial bioprosthetic heart valve according to an example of the present invention;
FIG. 5 is an expanded plan view showing a polyester rim coated for reinforcement at the curved bottom edge of a valve leaflet of an interventional aortic valve for interventional therapy of an artificial bioprosthetic heart valve according to an example of the present invention;
FIG. 6 is a perspective view of a metal stent of an interventional aortic valve for interventional therapy with an artificial heart valve according to an example of the present invention;
FIG. 7 is a perspective view of a metal stent of an interventional aortic valve for interventional therapy with an artificial heart valve according to another example of the present invention;
FIG. 8 is a schematic view of an interventional aortic valve structure for interventional therapy with an artificial heart valve according to another example of the present invention;
FIG. 9 is a schematic view of an interventional pulmonary valve structure according to yet another example of the present invention;
FIG. 10 is a perspective view of a stent structure of interventional pulmonary valves according to yet another example of the present invention;
FIG. 11 is a stent deployment plan view of an interventional pulmonary valve according to yet another example of the present invention.

### Detailed Description of the Invention

The present invention provides a connecting structure of a stent and a valve leaflet for an interventional aortic valve or an interventional pulmonary valve, and an interventional aortic valve and an interventional pulmonary valve using the connecting structure. The following detailed description illustrates specific embodiments with the understanding that the specific embodiments described herein are merely illustrative of the invention and are not intended to be limiting thereof. The connecting structure of the stent and the valve leaflet can be used for interventional pulmonary valves and interventional aortic valves.

### Example 1: interventional aortic valve

The interventional aortic valve has a "lower" end and an "upper" end. In the context of this application, the terms "lower" and "upper" are used interchangeably with the terms "inflow" and "outflow", respectively. Thus, for example, the lower end of the interventional aortic valve is its inflow end and the upper end of the interventional aortic valve is its outflow end.

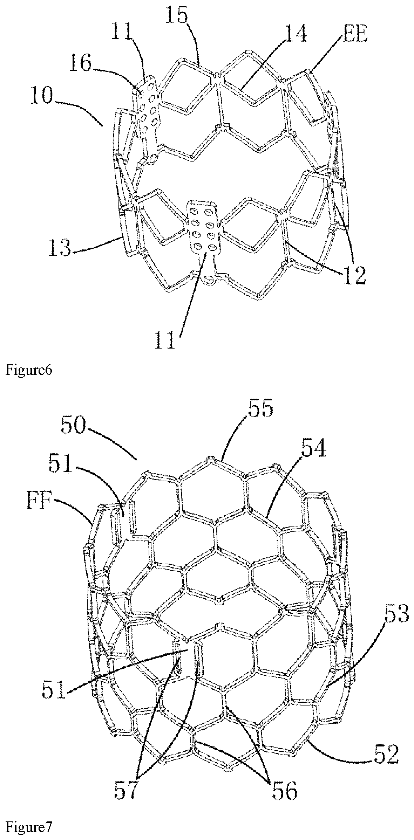

Referring to FIG. 2, there is shown a schematic view of an interventional aortic valve structure for interventional therapy of an artificial bioprosthetic heart valve according to an example of the present invention, comprising a stent 10 that can be radially compressible and in a slightly flaring shape after being expanded by a balloon, three fan-shaped valve leaflets 20 arranged on the inner side of the stent 10, the three fan-shaped valve leaflets 20 are respectively provided with a free edge 21, an arc-shaped bottom edge 22 and valve leaflet junction connecting parts 23 extending on the two sides (referring to FIG. 3). Referring further to FIG. 6, the stent is a metal mesh tube, wherein three connecting posts 11 are uniformly distributed on the metal mesh tube, six columns of axial supporting rods 12 are uniformly distributed among the three connecting posts 11, three rows of circumferential supporting rods 13, 14 and 15 transversely penetrating and extending are arranged between the connecting posts 11 and the six columns of axial supporting rods 12, and the lower first row of circumferential supporting rods 13 defines the inflow end of the stent 10, the second row of circumferential supporting rods 14 and the third row of circumferential supporting rods 15 spaced from the first row of circumferential supporting rods 13 define the outflow end of the stent. Each row of circumferential supporting rods is formed by connecting supporting rods EE with multiple constituent angles, each group of supporting rods EE is in the shape of a deformable V, the deformation angle is 0-90 degrees, and each group of circumferential supporting rods of the first row of circumferential supporting rods 13 and the second row of circumferential supporting rods 14 are arranged in parallel and opposite to the direction of each group of circumferential supporting rods of the third row of circumferential supporting rods 15 to form a plurality of grids which can be synchronously deformed. The stent made of the metal mesh tube can adapt to a use mode that an interventional aortic valve is longitudinally compressed and then expanded during interventional therapy. Further, the upper portion of the connection post 11 is provided with two rows of eight holes 16 for fixedly connecting the fan-shaped valve leaflets by sutures. In the plurality of grids of the stent 11, a coating membrane 17 is sewn between the first row of circumferential supporting rods 13 and the second row of circumferential supporting rods 14. The coating membrane on the body wall of the stent is sewn between the first row of circumferential supporting rods and the third row of circumferential supporting rods. In some cases, an outside coating membrane (not shown) is further sewn to the outside at the same location as desired. The material of the coating membrane can be animal-derived tissues or medical polymer materials which would occur to a person skilled in the art.

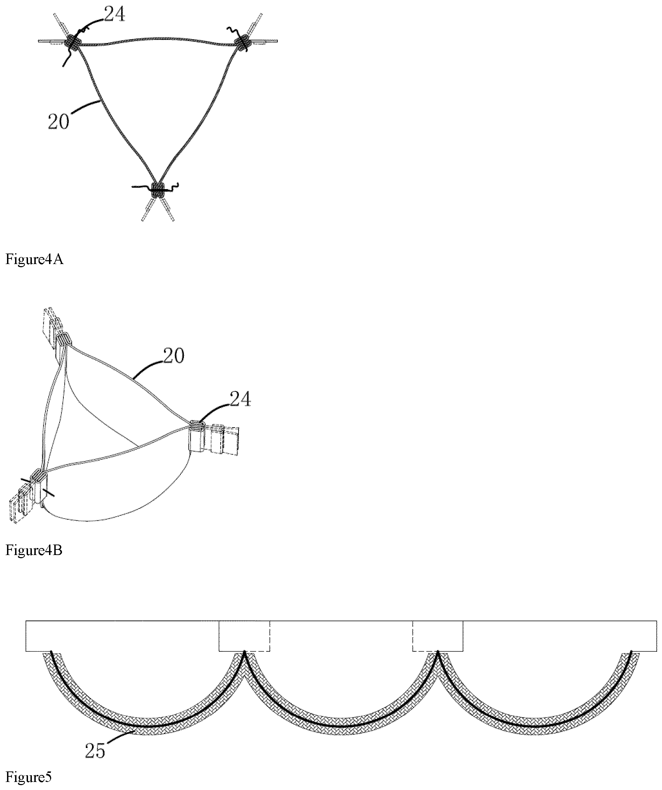

Referring again to FIGS. 2 and 4A-B, FIG. 5, the junction connecting part 23 of the fan-shaped valve leaflet 20 is first aligned in two pairs, and then folded outwardly two times each and then fitted into alignment with the fan-shaped valve leaflet 20 and secured by sutures, such that a section of cushioning portion 24 is formed at the root of the free edge of the valve leaflet, and the cushioning portion 24 is further secured to the connecting post 11 of the stent by sutures, and the arc-shaped bottom edge of the fan-shaped valve leaflet can also be coated with a reinforced coating membrane 25, and a coating membrane further sewn on the body wall of the stent through the coating membrane 25. Thus, an interventional aortic valve according to examples of the present invention is formed. Of course, if necessary, it is also possible to fold the junction connecting part only once to form the cushioning portion, but the cushioning portion is slightly lower in use effect than the cushioning effect of the two folds due to the fact that the rounded corners R naturally formed after the two folds are not formed.

When the valve leaflets of the interventional aortic valve are opened, due to the existence of the cushioning portion 24, the valve leaflets can be prevented from being rubbed or scratched by the metal stent in the opening and closing processes; and due to the fact that the connecting posts in the connecting structure are double rows of holes routing, the sutures at the seams of the junction connecting parts of the valve leaflets are fully fixed with the holes or the rectangular frame, stress concentration of the valve leaflets in the opening and closing processes can be avoided, use durability of the interventional aortic valve or the interventional pulmonary valve is improved, and a durable effect equivalent to that of a surgical valve is achieved.

### Example 2: interventional aortic valve

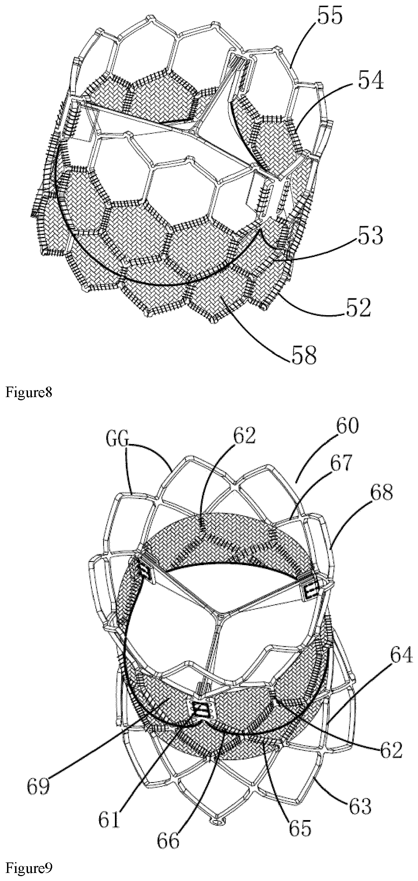

The interventional aortic valve of the example has a stent structure different from that of Example 1, and other structures are substantially the same as those of Example 1. In some cases, a larger outflow end and a larger stent height are required. Referring to FIGS. 7 and 8, in this example, the stent 50 is a metal mesh tube having three connecting posts 51 evenly distributed thereon, the stent is provided with four rows of transversely extending circumferential supporting rods 52, 53, 54 and 55 and a plurality of axial supporting rods 56 arranged between the circumferential supporting rods, wherein the lower first and second row of circumferential supporting rods 52, 53 define the inflow end of the stent, the third and fourth row of circumferential supporting rods 54, 55 define the outflow end of the stent, each row of circumferential supporting rods consists of a plurality of groups of angular supporting rods EE connected together, and each group of supporting rods is in a deformable V shape; the deformation angle is 0-90 degrees, the plurality of columns of axial supporting rods 56 and a plurality of groups of circumferential supporting rods are mutually connected to form a deformable honeycomb space. Wherein, the cellular grid in which the outflow end is defined is larger. Further, the upper portion of the connection post 11 is provided with two rows of rectangular frames 57 for fixedly connecting the fan-shaped valve leaflets by sutures. In the plurality of grids of the stent 51, a coating membrane 58 is sewn between the first row of circumferential supporting rods 52 and the third row of circumferential supporting rods 54. In some cases, an outer coating membrane (not shown) is also sewn to the outside of the coating membrane between the first row of circumferential supporting rods 52 and the second row of circumferential supporting rods 53, as desired. The material of the coating membrane can be animal-derived tissues or medical polymer materials which would occur to a person skilled in the art.

### Example 3: interventional pulmonary valve

Interventional pulmonary valves are commonly used for interventional therapy of the right ventricular outflow tract.

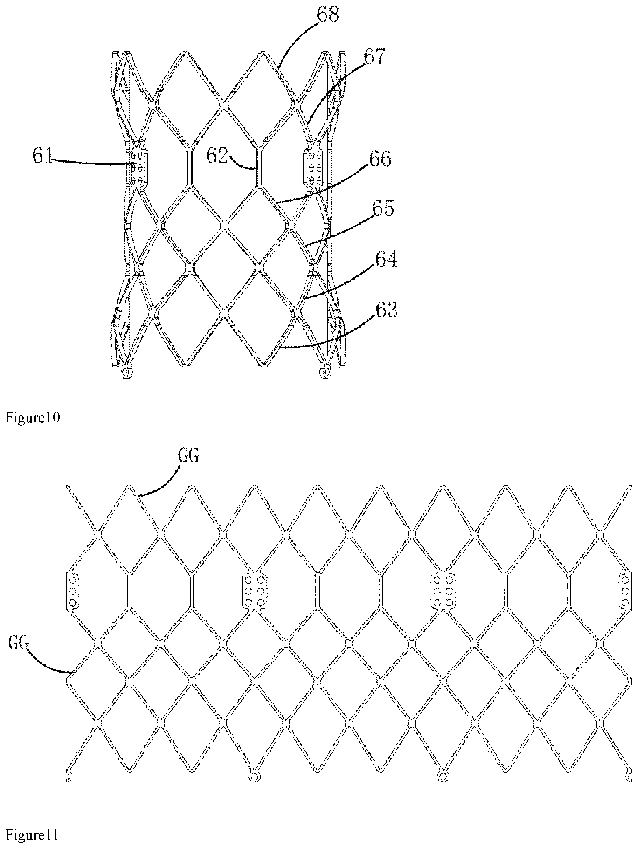

The interventional pulmonary valve of the present example is mainly different from the interventional aortic valves of Examples 1 and 2 of the present invention in that the shape of the stent is different. Referring to FIG. 9, the interventional pulmonary artery includes the stent and three fan-shaped valve leaflets, the shape of the stent further referring to FIGS. 10 and 11, the stent 60 has three connecting posts 61 and six rows of axial supporting rods 62 uniformly distributed on the three connecting posts, six columns of circumferential supporting rods 63, 64, 65, 66, 67 and 68 extending transversely are arranged at two ends of the connecting posts 61 and the axial supporting rods 62, and the lower first, second and third rows of circumferential supporting rods 63, 64 and 65 define inflow ends of the frame, the fourth, fifth and sixth rows of circumferential supporting rods 66, 67 and 68 define the outflow end of the stent, each row of circumferential supporting rods consists of a plurality of groups of angular supporting rods GG connected together, each set of supporting rods is in the form of a deformable V, the deformed angle is between 0 and 90 degrees, the plurality of columns of axial supporting rods 56 are interconnected with the fourth and fifth rows of circumferential supporting rods 66, 67 to form a deformable honeycomb grid, and the remaining rows of circumferential supporting rods form a rhombic grid. The coating membrane 69 on the body wall of the interventional pulmonary valve stent is sewn to the grid between the third row of circumferential supporting rods 65, the fifth row of circumferential supporting rods 67 and the three connecting posts 24.

The connecting post 61 of the interventional pulmonary valve of the example is of a double-row hole design, and the fan-shaped valve leaflets can be fixed to the stent in the same connection manner as in the Example 1.

The stent in all examples may be implemented as, but is not limited to, a cobalt-based alloy or nickel-titanium alloy or stainless steel material or other implantable alloy material stent or the like, and is not specifically limited thereto; the valve leaflet is an animal-derived tissue material or a medical polymer material, for example, any one of a porcine pericardium, a bovine pericardium or a sheep pericardium tissue material or any one of medical polymer materials, and is not specifically limited thereto. The suture is any one of medical polymer materials.

Finally, it should be noted that: the above examples are merely illustrative of the technical solutions of the present invention and are not intended to be limiting thereof; although the present invention has been described in detail with reference to the foregoing embodiments, those skilled in the art will appreciate that: the technical solutions of the above-mentioned embodiments can still be modified, or some or all of the technical features thereof can be equivalently replaced; these modifications or replacements do not cause the essence of the corresponding technical solutions to deviate from the scope of the technical solutions of the embodiments of the present invention.

## Claims

1. A connecting structure of a stent and a valve leaflet for interventional pulmonary valves and interventional aortic valves, wherein the stent is a metal mesh tube, the valve leaflets are three fan-shaped valve leaflets arranged on the inner side of the stent, each of the three fan-shaped valve leaflets is provided with a free edge, an arc-shaped bottom edge and valve leaflet junction connecting parts extending on two sides, **characterized in that** three connecting posts are uniformly distributed on the metal mesh tube, and the junction connecting parts on the two sides of each valve leaflet are folded on the inner side of each connecting post to form a cushioning portion, and then are connected and fixed to the connecting posts through sutures.

2. The connecting structure of the stent and the valve leaflet for interventional pulmonary valves and interventional aortic valves of claim 1, **characterized in that** double columns of holes or double columns of rectangular frames are arranged on the each connecting post, the number of the holes is four to eight, and the number of the rectangular frames is two or four.

3. The connecting structure of the stent and the valve leaflet for interventional pulmonary valves and interventional aortic valves of claim 2, **characterized in that** the junction connecting part is folded once or twice to form the cushioning portion and connected and fixed to the hole or the rectangular frame of the connecting post through sutures.

4. The connecting structure of the stent and the valve leaflet for interventional pulmonary valves and interventional aortic valves of claim 1, **characterized in that** the material of the stent is an implantable alloy material, the implantable alloy material is a cobalt-based alloy, a nickel-titanium alloy or a stainless steel material, and the material of the valve leaflet is an animal-derived tissue material or a medical polymer material.

5. An interventional pulmonary valve employing the connecting structure of the stent and the valve leaflet of any one of claims 1-4, comprising a stent being radially compressible and self-expandable with two ends in a uniform cylindrical flaring shape, three fan-shaped valve leaflets arranged on the inner side of the stent, wherein the three fan-shaped valve leaflets are respectively provided with a free edge, an arc-shaped bottom edge and valve leaflet junction connecting parts extending on the two sides, **characterized in that** the stent is a metal mesh tube, three connecting posts are uniformly distributed on the metal mesh tube; and the junction connecting parts on the two sides of each valve leaflet are folded on the inner side of each connecting post to form a cushioning portion, then are connected and fixed to the connecting posts through sutures, and a coating membrane is arranged on the wall body of the stent.

6. The interventional pulmonary valve of claim 5, **characterized in that** the stent is provided with a plurality of columns of axial supporting rods arranged between the connecting posts, six rows of transversely extending circumferential supporting rods are arranged between the connecting posts and the axial supporting rods, the lower first, second and third rows of circumferential supporting rods define an inflow end of the stent, the fourth, fifth and sixth rows of circumferential supporting rods define an outflow end of the stent, and each row of circumferential supporting rods consists of a plurality of groups of angular supporting rods connected together; each group of supporting rods is in the shape of a deformable V, the deformation angle is 0-90 degrees, each row of circumferential supporting rods and a plurality of columns of axial supporting rods form a plurality of rows of rhombic or honeycomb grids, and the coating membrane on the body wall of the stent is sewn to the grids in the middle row of the stent.

7. The interventional pulmonary valve of claim 5, **characterized in that** an angle between the outer edge of the balloon-expanded stent and the axis thereof is between 0°and 30°.

8. An interventional aortic valve employing the connecting structure of the stent and the valve leaflet of any one of claims 1-4, comprising a stent that can be radially compressible and in a slightly flaring shape after being expanded by a balloon, three fan-shaped valve leaflets arranged on the inner side of the stent, wherein the three fan-shaped valve leaflets are respectively provided with a free edge, an arc-shaped bottom edge and valve leaflet junction connecting parts extending on the two sides, **characterized in that** the stent is a metal mesh tube, and three connecting posts are uniformly distributed on the metal mesh tube; and the junction connecting parts on the two sides of each valve leaflet are folded on the inner side of each connecting post to form a cushioning portion, then are connected and fixed to each of the connecting posts through sutures, and a coating membrane is arranged on the body wall of the stent.

9. The interventional aortic valve of claim 8, **characterized in that** the stent is provided with a plurality of columns of axial supporting rods arranged between the connecting posts, three rows of transversely extending circumferential supporting rods are arranged between the connecting posts and the axial supporting rods, the lower first row of circumferential supporting rods define an inflow end of the stent, the second row of circumferential supporting rods and the third row of circumferential supporting rods spaced from the first row define an outflow end of the stent, and each row of circumferential supporting rods consists of a plurality of groups of angular supporting rods connected together; each group of supporting rods is in the shape of a deformable V, the deformation angle is 0-90 degrees, each group of circumferential supporting rods in the first row and the second row are arranged in parallel and opposite to the direction of each group of circumferential supporting rods in the third row, and the coating membrane on the body wall of the stent is sewn between the first row of circumferential supporting rods and the second row of circumferential supporting rods.

10. The interventional aortic valve of claim 8, **characterized in that** the stent is provided with four rows of transversely extending circumferential supporting rods and a plurality of columns of axial supporting rods arranged between the circumferential supporting rods, wherein the lower first and second row of circumferential supporting rods define an inflow end of the stent, the third and fourth row of circumferential supporting rods define an outflow end of the stent, each row of circumferential supporting rods consists of a plurality of groups of angular supporting rods connected together, and each group of supporting rods is in a deformable V shape; the deformation angle is 0-90 degrees, the plurality of columns of axial supporting rods and the plurality of groups of circumferential supporting rods are mutually connected to form a honeycomb space, and the coating membrane on the body wall of the stent is sewn between the first row of circumferential supporting rods and the third row of circumferential supporting rods.

11. The interventional aortic valve of claim 8, **characterized in that** a coating membrane is sewn between the first row of circumferential supporting rods and the second row of circumferential supporting rods outside the body wall of the stent.

12. The interventional aortic valve of claim 8, **characterized in that** the angle between the outer edge of the balloon-expanded stent and the axis thereof is between 0°and 30°.
